(19) Europäisches Patentamt
European Patent Office
Office européen des brevets

(11) EP 2 168 593 A1

(12) EUROPEAN PATENT APPLICATION

(43) Date of publication:
**31.03.2010 Bulletin 2010/13**

(21) Application number: **09013789.4**

(22) Date of filing: **31.08.1999**

(51) Int Cl.:
***A61K 39/00*** *(2006.01)* ***A61K 9/16*** *(2006.01)*
***A61K 9/51*** *(2006.01)* ***A61K 39/10*** *(2006.01)*
***A61K 9/00*** *(2006.01)*

(84) Designated Contracting States:
**AT BE CH CY DE DK ES FI FR GB GR IE IT LI LU MC NL PT SE**

(30) Priority: **01.09.1998 US 98759 P**

(62) Document number(s) of the earlier application(s) in accordance with Art. 76 EPC:
**99940438.7 / 1 107 782**

(71) Applicant: **Merrion Research III Limited**
**Dublin 2 (IE)**

(72) Inventor: **Brayden, David, James**
**Blackrock**
**County Dublin (IE)**

(74) Representative: **Ryan, Anne Mary et al**
**Anne Ryan & Co.**
**60 Northumberland Road**
**Ballsbridge**
**Dublin 4 (IE)**

Remarks:
This application was filed on 03-11-2009 as a divisional application to the application mentioned under INID code 62.

# (54) Oral vaccine compositions

(57) Oral vaccine formulations are disclosed having microparticles sized such that at least 50% of the microparticles are less than 5 μm, preferably less than 3 μm, the microparticles containing antigen entrapped or encapsulated, such as by a solvent evaporation method, by a biodegradable polymer, such as poly (D,L-lactide-co-glycolide). Additionally, oral vaccine formulations are disclosed having nanoparticles sized such that at least 50% of the microparticles are less than 600nm, preferably less than 500nm, the nanoparticles containing antigen entrapped or encapsulated, such as by a coacervation method, by a biodegradable polymer, such as poly (D,L-lactide-co-glycolide). Protective vaccine formulations containing the *B. pertussis* antigens PTd or a combination of PTd and FHA are provided.



Printed by Jouve, 75001 PARIS (FR)

## Description

Technical Field

The present invention relates to oral vaccine formulations. In particular, the present invention relates to oral microparticulate or nanoparticulate vaccine formulations comprising antigens entrapped by or encapsulated within polymer particles.

Background Art

Controlled release antigen delivery systems have attracted considerable interest in the continuing search for vaccine carriers. The effectiveness of polymer matrices in the sustained release of antigen was first demonstrated in 1979 with the entrapment of bovine serum albumin in a non-degradable ethylene-vinyl acetate copolymer pellet for subcutaneous implantation [Preis et al., J. Immunol. Methods 28, 193-197 (1979)]. This composition induced an antibody response for six months after administration and gave antibody levels similar to two injections of the same total amount of antigen in complete Freund's adjuvant.

More recently aluminium salts (*see* for example WO 94/15636 (CSL Ltd.)) and biodegradable polymers such as poly (L-lactide) (hereinafter PLA) and poly (DL-lactide-*co*-glycolide) (hereafter PLGA) have been used as carriers for vaccine antigens. WO 95/11008 (Genentech Inc.) discloses the use of PLGA microspheres for encapsulating an antigen in which the ratio of lactide to glycolide in the microspheres ranges from 100:0 to 0:100 weight percent, the inherent viscosity of the PLGA polymers ranges from 0.1 - 1.2 dl/g and the median diameter of the microspheres ranges from 20 - 100 μm. The antigen can be continuously released from the microspheres over an extended period in a triphasic pattern. A method for encapsulating antigens in microspheres is also disclosed.

Eldridge et al., J. Controlled Release 11, 205-214 (1990) report that the oral administration of biodegradable PLA or PLGA microspheres containing the staphylococcal enterotoxin B (SEB) vaccine are absorbed into the Peyer's patches of the small intestine. Uptake is restricted to particles ≤ 10 μm in diameter. The majority of microspheres < 5 μm were observed to be transported to systemic lymphoid tissue (such as the spleen) where the released antigen stimulated a serum antibody response. The majority of those particles > 5 μm were found to be retained in the Peyer's patches. EP 0 266 119 (The UAB Research Foundation & Southern Research Institute) teaches an oral composition comprising a bioactive agent, such as an antigen, encapsulated in a biodegradable polymer excipient to form a microcapsule less than or equal to 10 μm that is capable of being taken up selectively by the Peyer's patch. Similarly, EP 0 333 523 (The UAB Research Foundation & Southern Research Institute) and EP 0 706 792 divided therefrom, teach compositions for delivery of a bioactive agent, such as an antigen, to the mucosally associated lymph tissue (MALT), comprising microcapsules having sizes between 1-5 and 5-10 μm for selective absorption and retention in MALT.

EP 0 686 030 (Gesellschaft zur Forderung der Industrieorientierten Forschung) teaches a method of potentiating an immune response by embedding a model antigen in a biodegradable biopolymer and injecting it in the form of a dispersion in order to trigger a humoral and cellular response. In this instance PLGA entrapped antigens were shown to elicit long lasting T helper, antibody and cytotoxic T cell responses.

Moore et al., Vaccine 18 1741-1749 (1995) discloses that HIV gp 120 entrapped in PLGA solvent evaporated microspheres can induce cytolytic activity (CTL) in mice splenic T cells upon nasal, s.c. or i.p. administration. For this antigen, anti-HIV specific CD4+ and CD8+ T cells were induced leading to induction of $T_H1$ cells and CTL respectively. In the case of i.p. ovalbumen (OVA) immunisation, Maloy et al., Immunology 81, 661-667 (1994) disclose that a single s.c. immunisation with OVA-PLGA microspheres primed significant OVA-specific responses and strong OVA-specific CTL responses were found after i.p immunisation in mice. Newman et al., J. Controlled Release, 54, 49-59 (1998) disclose the use of OVA peptide encapsulated in PLGA microspheres for inducing a $T_H1$ type immune response in mice after s.c. delivery.

Distinction between the types of immune response in terms of $T_H1$ (cell-mediated) and $T_H2$ (humoral / antibody) type responses is important for protection against infectious diseases induced by intracellular pathogens or extracellular toxins respectively. The division of CD4+ lymphocytes into $T_H1$ and $T_H2$ according to antibody sub-class and cytokine profile has led to attempts to classify adjuvants accordingly. For instance, aluminium hydroxide (also referred to as alum) is considered to have a higher capacity for inducing $T_H2$ rather than $T_H1$ immunity [*see*, e.g., Men et al., Vaccine 13, 683-689 (1995)]. US 5,417,986 (US Army) describes the loading of PLGA microspheres with antigens such as CFA (complete Freund's adjuvant) and HepB sAg (hepatitis B surface antigen) and injected to give both antibodies and T cell proliferation in animals.

Review of the above cited references and other literature in the area shows that there is no general method for predicting or anticipating the nature of the immune response induced by an antigen in combination with a given adjuvant.

With respect to *Bordetella pertussis,* the fimbrae, filmentous hemaglutinin (FHA), inactivated pertussis toxin (PTd) and pertactin antigens have all been entrapped in PLGA microspheres, administered individually by a variety of routes and have been shown to protect against infection in response to challenge in a mouse model *of pertussis* (*see,* e.g., Shahin et al., Infection and Immunity 63, 1195-1200 (1995); Jones et al., Infection and Immunity 64, 489-494 (1996*);* Cahill et al., Vaccine 13, 455-462 (1995)). WO 93/21950 (Roberts and Dougan) teaches that the antigens FHA and pertactin are immunogenic as a mixture or when entrapped in PLGA and delivered to mucosal sites. Singh et al., Vaccine 16, 346-352 (1998) describe that two antigens entrapped simultaneously in the same polymer particles can induce antibody responses to each agent in rats after parenteral delivery. There is evidence that the mouse model of aerolised *pertussis* infection correlates with *pertussis* vaccine efficacy in children [Mills et al., Infection and Immunity 66, 594-602 (1998)] and that $T_H1$ cells play an important role in bacterial clearance [Mills et al., Infection and Immunity 61, 399-410 (1993)]. Further work by Ryan et al., Immunology 93, 1-10 (1998) indicates that the long-term protective immunity of a potent whole cell *pertussis* vaccine in children is largely mediated by $T_H1$ cells. Acellular *pertussis* vaccines appear to involve a mixed population of $T_H1$ and $T_H2$ cells and their long term efficacy is unknown.

Despite the above-mentioned prior art, the ability to predict and control the type of immune response produced by a given vaccine formulation remains a goal central to immunology research. This is particularly true given the variation from disease to disease of the relative importance of $T_H1$ and $T_H2$ components of the immune response. For example, $T_H1$ response can assist in cytotoxic T cell activity which is important in clearances of viruses, intracellular pathogens and some cancers.

Therefore it is an object of the present invention to provide an oral vaccine that is capable of providing protective immunity against a particular agent such as an infectious or pathogenic agent. It is an additional object to provide an oral protective vaccine formulation which contains microparticles having at least one antigen entrapped or encapsulated by a biodegradable polymer.

Further objects of the present invention include an improved composition for use in the preparation of a *B. pertussis* vaccine and a method for the vaccination against *B. pertussis.*

## Disclosure of Invention

It has now been surprisingly found that an effective, protective immune response can be induced by oral administration of microparticles and/or nanoparticles comprising antigen(s) entrapped by or encapsulated in a biodegradable polymer using a suitable combination of polymer type, loading method and size.

Accordingly, the present invention provides a method of inducing a protective immune response against an agent such as an infectious agent, pathogenic agent or a cancer agent, comprising orally administering to a subject, such as a mammal and preferably a human, microparticles sized such that at least 50% of the microparticles are less than 5 μm, preferably less than 3 μm, the microparticles comprising an antigen(s) entrapped or encapsulated by a biodegradable polymer. A vaccine formulation for oral administration comprising microparticles sized such that at least 50% of the microparticles are less than 5 μm, preferably less than 3 μm, the microparticles comprising antigen(s) entrapped or encapsulated by a biodegradable polymer is also provided.

Additionally, the present invention provides a method of inducing a protective immune response against an agent such as an infectious agent, pathogenic agent or a cancer agent, comprising orally administering to a subject, such as a mammal and preferably a human, nanoparticles sized such that at least 50% of the nanoparticles are less than 600 nm, preferably less than 500 nm, the nanoparticles comprising an antigen entrapped or encapsulated by a biodegradable polymer. A vaccine formulation for oral administration comprising nanoparticles sized such that at least 50% of the nanoparticles are less than 600nm preferably less than 500nm, the nanoparticles comprising antigen(s) entrapped or encapsulated by a biodegradable polymer is also provided.

The present invention also provides a method of providing protective immunity against *B. pertussis,* comprising orally administering to a subject microparticles sized such that at least 50% of the microparticles are less than 5 μm, preferably less than 3μm, the microparticles comprising at least one *B. pertussis* antigen entrapped or encapsulated by a biodegradable polymer. Also, the present invention provides a method of providing protective immunity against *B. pertussis,* comprising orally administering to a subject nanoparticles sized such that at least 50% of the nanoparticles are less than 600nm, preferably less than 500 nm, the nanoparticles comprising at least one *B. pertussis* antigen entrapped or encapsulated by a biodegradable polymer. Preferably, the microparticles or nanoparticles contain at least two B. *pertussis* antigens, such as inactivated *B. pertussis* toxin or FHA.

Preferably, the antigen(s) is capable of eliciting an immune response upon administration, the antigen being entrapped and / or encapsulated within a biocompatible, biodegradable polymer carrier material. Preferably, the method for entrapping and / or encapsulating the antigen within the polymer carrier material is a solvent evaporation based process or a coacervation process.

The present invention further relates to a method for the prevention of *B. pertussis* which method comprises administration of a composition comprising at least one *B. pertussis* antigen such as inactivated *B. pertussis* toxin and/or FHA encapsulated in poly (DL-lactide-co-glycolide) particles, wherein encapsulation of the *B. pertussis* antigen in poly (DL-lactide-*co*-glycolide) particles is carried out by solvent evaporation or coacervation and wherein administration is oral.

Brief Description of Drawings

Figure 1 shows the $T_H1/T_H2$ responses following oral immunisation with KLH entrapped in PLGA. Groups of 4 mice received oral inoculations with 100μg of KLH encapsulated in PLGA microparticles (KLH-PLGA) or 100 μg soluble KLH in combination with empty PLGA microparticles (KLH + PLGA). Mice were immunised twice (0 and 4 weeks) and sacrificed two weeks later. Spleen cells from individual mice were stimulated with 0.03 - 20 μg/ml of KLH or with medium alone. After 3 days culture supernatants were tested for IL-5 and IFN-γ by specific immunoassays. Each bar represents the mean response for 4 mice in each group. Note the difference in the scale for IL-5 (pg/ml) and IFN-γ (ng/ml);

Figure 2 shows the results of the T cell proliferation assay according to Example 5. 4 groups of balb/c mice were immunised by oral gavage at week 0 and week 4 with 100 μg PTd entrapped in PLGA (PTd in PLG); with 100 μg soluble PTd in combination with empty PLGA microparticles (soluble PTd + ePLG); with 100 μg soluble PTd (soluble PTd) and with empty PLGA microparticles (empty PLG). The mice were sacrificed at week 6 and assayed for T cell proliferation in 4 day cultures by [$^3$H]-thymidine incorporation;

Figure 3 shows the serum antibody titres to PTd following oral administration, as described in Example 5, of 100 μg PTd entrapped in PLGA (PTD in PLG); 100 μg soluble PTd in combination with empty PLGA microparticles (soluble PTd + ePLG); 100 μg soluble PTd (soluble PTd) and empty PLGA microparticles (empty PLG);

Figures 4 and 5 show the cytokine analysis from splenic T cells according to Example 5. Four groups of balb/c mice were immunised by oral gavage at week 0 and week 4 with 100 μg PTd entrapped in PLGA (PTD in PLG); with 100 μg soluble PTd in combination with empty PLGA microparticles (sPTd + empty PLG); with 100 μg soluble PTd (soluble PTd) and with empty PLGA microparticles (empty PLG) and assayed at week 6. Spleen cells from individual mice were stimulated with inactivated PT (iPT), *B. pertussis* (B. pert) and with the positive control anti-CD3-antibody / phorbol 12-myristate-13 acetate (PMA/aCD3). After 3 days, culture supernatants were tested for IL-5 and IFN-γ by specific immunoassays; and

Figure 6 shows a plot of $Log_{10}$ CFU counts *per* lung *versus* Days after challenge for the following lung homogenate culture samples from the respiratory challenge study given in Example 6: control PLG (mice immunised with empty PLGA microparticles), PTd-PLG (mice immunised with 100 μg of PTd entrapped in PLGA microparticles), PTd SOLUTION (mice immunised with 100 μg of PTd in solution) and PTd-FHA-PLG (mice immunised with 100 μg of each of PTd and FHA entrapped in PLGA microparticles). The mice were dosed three times with an interval of 4 weeks between each dosing and presented with an aerosol challenge two weeks after the third dose.

Figure 7 shows a plot of $Log_{10}$ CFU counts *per* lung *versus* Days after challenge for the following lung homogenate culture samples from the respiratory challenge study given in Example 8: CONTROL (mice immunised with empty PLGA coacervated nanoparticles), SOLUBLE PTd + FHA (mice immunised with 100 μg of PTd and FHA in solution) and PTd-FHA-PLG (mice immunised with 100 μg of each of PTd and FHA entrapped in PLGA coacervated nanoparticles). The mice were dosed three times with an interval of 4 weeks between each dosing and presented with an aerosol challenge two weeks after the third dose.

While vaccine formulations which comprise antigens loaded onto polymer particle are known in the prior art it has now been found that the choice of biocompatible carrier material, the method of loading of the biologically active agent (ie. the method for adsorbing and / or encapsulating the biologically active agent onto and / or within the biocompatible, biodegradable polymer material) and the route of administration are all contributing factors in determining the nature of the immune response produced. By a suitable combination of the above listed determinants a composition may be prepared which elicits a polarised immune response. Polarisation of the immune response may be characterised by determination of the relative proportions of $T_H1$ and $T_H2$ indicators, typically cytokines such as IFN-γ, TNF, IL-2 or IL-12 and IL-5, IL-4, IL-6 or IL-10 specific to $T_H1$ and $T_H2$ responses, respectively.

Biologically active agents suitable for the practice of the present invention are typically antigens capable of eliciting a polarised $T_H1$ immune response, a polarized $T_H2$ response or a mixed $T_H1/T_H2$ response upon administration. Preferred antigens include those selected from the list comprising PTd, inactivated pertussis toxin or pertactin; FHA, filamentous hemaglutinin; TT, tetanus toxoid; HIV gp-120; hepatitis B surface antigen; DT, diptheria toxoid; HSV, herpes simplex type 1; HPV, human papilloma virus; polio; influenza epitopes; H. *pylori;* shigella; chlorea; salmonella; rotavirus; RSV, respiratory virus; yellow fever; hepatitis A and C; meningoccoccal types A - C; pneumococcal; parasites such as leischmania; mycobacteria such as tuberculosis; and cancer vaccine antigens.

As used herein, the term "protective immunity" in refers to at least 75% clearance, more preferably 90% clearance of the challenging agent, such as an infectious agent, from the subject preferably within 3 weeks after the introduction of the challenging agent, more preferably within 2 weeks, most preferably within days.

As used herein, the term "pharmaceutically effective amount" refers to the amount of antigen required to elicit a protective immunity response to that antigen. For instance, in mice, protective immunity is achieved with an amount of a *B. pertussis* antigen(s) in the range of up to 100 μg for each antigen given orally in multiple doses, such as 3 doses, or in a single dose.

As used herein, references to the sizes of microparticles and/or nanoparticles refer to sizes as determined by visual assessment of scanning electron micrographs and/or, where indicated, laser light diffractometry.

It has been found that the choice of biocompatible, biodegradable polymer material used as a carrier for the antigen and the method of loading the carrier with antigen are important in defining the nature of immune response achieved. Preferably the biocompatible, biodegradable polymer material is a copolymer of lactic acid and glycolic acid, such as 50:50 poly (D,L-lactide-co-glycolide), poly (lactide-co-glycolide), and enantiomers thereof or a polymer of lactic acid, such as poly (lactide) and enantiomers thereof. The antigen can be loaded by either a solvent evaporation type process or a spray drying process, preferably a solvent evaporation type process. Further details of the loading processes that can be used are given in the Examples below.

Modes for Carrying Out the Invention

As will be further appreciated from the examples below the nature of the immune response elicited by antigen loaded polymer particles does not depend on a single factor, but is governed by a combination of a number of factors.

***Examples***

All percentages are by weight (w/w) unless otherwise stated. The following abbreviation are used throughout the examples: KLH, keyhole limpet hemacyanin; PTd, inactivated pertussis toxin; FHA, filamentous hemaglutinin; PLA, poly lactide; PLGA, poly lactide-co-glycolide; DCM, dichloromethane; PVA, poly vinyl alcohol; PBS, phosphate buffer solution.

Example 1

Preparation of KLH-PLGA microparticles using a solvent evaporation method.

A polymer solution of PLGA [poly (D,L-lactide-co-glycolide), 50:50; i.v. = 0.94 dl / g; supplied by Boehringer Ingelheim] in dichloromethane (10 % PLGA in 10 ml DCM) was prepared two hours prior to use and subsequently chilled 30 minutes prior to use. The antigen, KLH (supplied by Calbiochem as a powder), was prepared as an aqueous solution (5.1 mg KLH in 1 ml water) containing 2 % PVA. A first water-in-oil emulsion was prepared by adding the antigen solution to the polymer solution and homogenising for 1 min. at 24,000 rpm on ice. This first emulsion was poured slowly into an aqueous solution of PVA (40 ml, 3 % PVA) forming a second water-oil-water emulsion and homogenisation was continued for 2 min. with a 15 sec. break [1 min.; 15 sec. break; 1 min.]. The resulting emulsion was stirred for 2 hours to evaporate the dichloromethane. The antigen-loaded particles (75% yield) were collected by centrifugation (10,000 rpm for 15 min).

The morphology and the particle size of the KLH-PLGA particles were examined by scanning electron microscopy (SEM) using a Leica Cambridge S360. Samples were mounted on stubs, gold coated and scanned at magnifications of x3,000 - 10,000. Particle size assessment by SEM was carried out by dividing the micrographs at the 5,000 or 10,000 magnification into different fields and counting the number of particles greater and less than 3 microns and 5 microns. Particle size determination was carried out by laser diffractometry using a Malvern Mastersizer S Ver. 2.14. The microparticles were suspended in filtered 0.1 % Tween 20, sonicated for 5 minutes and analysed with continuous stirring. KLH-PLGA particles prepared as detailed above were found to have a smooth spherical appearance and a D50% of 2.5 μm. By SEM, it could be seen that at least 50 % of the particles had a diameter less than 5 microns.

The loading of microparticles with antigen was determined by digesting 10 mg of loaded microparticles in 3 ml of 5 % SDS / 0.1 M NaOH for up to 60 hours with continuous shaking at room temperature. The particles were completely digested during this period. The pH of the solution was adjusted to pH 11.2 with 0.1 M HCl and protein content was determined using a Bicinchoninic acid (BCA) protein assay kit. Equivalent control particles containing no antigen were also digested. The loading was calculated as follows:

$$\text{Actual loading } (\mu g/mg) = \frac{\text{concentration in sample } (\mu g/ml) \text{ x total volume digested (ml)}}{\text{weight of particles (mg)}}$$

$$\text{\% entrapment efficiency} \frac{\text{actual loading } (\mu g/mg) \text{ x } 100}{\text{theoretical loading } (\mu g/mg)}$$

where the theoretical loading is calculated from the amount of antigen added to the formulation divided by the amount of polymer used.

KLH-PLGA particles prepared according to the present example were found to have a loading of 3.1 μg antigen / mg particles, giving an entrapment efficiency of 94 %.

The *in vitro* release of antigen from the loaded particles was determined as follows: antigen loaded microparticles and control microparticles (prepared in a similar manner, but containing no antigen) were accurately weighed and dispersed in PBS containing 0.02 % sodium azide as a bacteriostatic agent. Samples were immersed in a water bath at 37°C and shaken continuously. At appropriate time intervals, 2.2 ml aliquots were removed with a syringe, filtered and the protein content measured in duplicate by BCA assay. KLH-PLGA particles prepared according to the present example were found to release 80 % of loaded antigen after 1 hour and 100 % of loaded antigen after 24 hours.

The procedure detailed above was repeated to form a second batch of KLH-PLGA microparticles. This second batch of microparticles appeared smooth and spherical under SEM, with at least 50 % of the particles less than 5 microns, the D50% was determined to be 2.2 μm; the loading was found to be 3.5 μg/mg representing 94 % entrapment efficiency; and 76 % of the antigen was determined to be released after 1 hour, with 90 % being released after 24 hours.

Antigen loaded microparticles obtained from these two batches were pooled together for an immunogenicity study in mice as discussed in Example 4 below.

Example 2

Preparation of PTd-PLGA microparticles using a solvent evaporation method.

Using a method substantially the same as that described in Example 1 above, PTd (supplied by Katetsuken) loaded PLGA particles were prepared. The polymer solution was 6.7 % PLGA in 15 ml DCM and the antigen solution was 744 μg PTd in 2 ml water containing 0.9 % PVA. The first water-in-oil emulsion was poured into 80 ml aqueous PVA (3 % PVA) to form the water-oil-water emulsion. The emulsion was left over night to evaporate the DCM. After collection (88% yield), the microparticles were washed with chilled autoclaved water (30 ml).

Characterisation of these particles, identified as PTd-1 in Table 1 below, showed that the microparticles formed were smooth and spherical in appearance with at least 50 % of the particles less than 5 microns in diameter. Laser light diffractometry showed that the particles had a D50% of 2.5 μm. The microparticles were loaded with antigen at 0.12 μg/mg, representing an entrapment efficiency of 15 %.

The *in vitro* release of PTd loaded microparticles was determined according to the following method: 30 mg of microparticles were dispersed in PBS (4.0 ml) containing 0.02 % sodium azide. The sample was placed in a water bath at 37 °C and shaken continuously. At appropriate time intervals the sample was removed from the water bath and centrifuged to pellet the particles. The supernatant was removed and the protein content was determined in duplicate. Three ml of fresh PBS was added to the microparticles to maintain sink conditions and the incubation was continued. PTd-PLGA particles prepared according to the present example (PTd-1) were found to release 22 % of loaded antigen after 1 hour and 56 % of loaded antigen after 24 hours followed by biphasic release over 20 days.

Additional batches of PTd-PLGA microparticles were made following substantially the same procedure as given above using quantities of the various components as summarised in Table 1 below. In batches PTd-2 through PTd-6, no PVA was added to the initial antigen solution and 40 ml chilled autoclaved water was used to wash the recovered microparticles. In each case, the resulting antigen loaded microparticles were found to be smooth and spherical in appearance with at least 50 % of the particles less than 5 microns in diameter.

| Table 1 | | | | | | | | | | |
|---|---|---|---|---|---|---|---|---|---|---|
| Batch No. | PTd (μg) | Aq. Vol (ml) | % PLG A | DC M (ml ) | 3% PVA (ml) | Load (μg/ mg) | % EE | D50 % (μm) | 1 hr (% ) | 24 hr (%) |
| PTd-1 | 744 | 2 | 6.7 | 15 | 80 | 0.12 | 15 | 2.5 | 22 | 56 |
| PTd-2 | 153 6 | 1.0 | 4 | 10 | 40 | 1.2 | 31 | 3.0 | 30 | |
| PTd-3 | 276 0 | 1.5 | 4 | 20 | 80 | 1.3 | 33 | 3.3 | * | * |
| PTd-4 | 313 0 | 1.5 | 4 | 20 | 80 | 1.3 | 34 | 2.4 | * | * |
| PTd-5 | 214 0 | 2.0 | 4 | 20 | 80 | 1.1 | 42 | 3.2 | * | * |
| PTd-6* | - | - | - | - | - | - | - | - | 17 | 21 |
| 3% PVA is the volume of PVA solution to which the antigen / PLGA water-in-oil emulsion is added; Load is the antigen loading of the microparticles; %EE is the % entrapment efficiency; D50% is the average diameter of the microparticles; 1 hr is the antigen released after 1 hour; 24 hr is the antigen released after 24 hours. *The loaded microparticles obtained from PTd3, PTd-4 and PTd-5 were pooled for antigen release assay and i.p. protection study (*see* Example 7 below). | | | | | | | | | | |

Example 3

Preparation of FHA-PLGA microparticles using a solvent evaporation method.

A procedure substantially similar to that used in Example 2 was employed for the preparation of FHA-loaded PLGA microparticles. Two batches of FHA-PLGA microparticles were prepared. For these two batches (FHA-1 and FHA-2 in Table 2 below) the polymer solution was 4 % PLGA in 20 ml DCM and the antigen solution was 0.87 μg FHA in 2 ml water containing no PVA. The first water-in-oil emulsion was poured into 80 ml aqueous PVA (3 % PVA) to form the water-oil-water emulsion. The characteristics of these two batches are given in Table 2 below. FHA-1 and FHA-2 were pooled (the pooled microparticles are labelled FHA-3 in Table 2) for antigen release determination and i.p. protection studies (*see* Example 6 below). SEM analysis showed the FHA-1 and FHA-2 microparticles to be smooth and spherical in nature with at least 50 % of the particles less than 5 microns in diameter.

| Table 2 | | | | | |
|---|---|---|---|---|---|
| Example No. | Loading (mg/mg) | % EE | D50% (μm) | 1 hr (%) | 24 hr (%) |
| FHA-1 | 0.94 | 87 | 3.0 | * | * |
| FHA-2 | 1.09 | 100 | 4.3 | * | * |
| FHA-3* | - | - | - | 25 | 49 |

Example 4

Preparation of antigen entrapped or encapsulated in nanoparticles

An aqueous solution (A) of a polymer, surface active agent, surface stabilising or modifying agent or salt, or surfactant preferably a polyvinyl alcohol (PVA) or derivative with a % hydrolysis 50 - 100% and a molecular weight range 500 - 500,000, most preferably 80-100% hydrolysis and 10,000-150,000 molecular weight, is introduced into a vessel. The mixture (A) is stirred under low shear conditions at 10- 2000 rpm, preferably 100-600 rpm. The pH and/or ionic strength of this solution may be modified using salts, buffers or other modifying agents. The viscosity of this solution may be modified using polymers, salts, or other viscosity enhancing or modifying agents.

A polymer, preferably poly(lactide-co-glycolide), polylactide, polyglycolide or a combination thereof or in any enantiomeric is dissolved in water miscible organic solvents to form organic phase (B). Most preferably, a combination of acetone and ethanol is used in a range of ratios from 0:100 acetone: ethanol to 100: 0 acetone: ethanol depending upon the polymer used. Additional polymer(s), peptide(s) sugars, salts, natural/biological polymers or other agents may also be added to the organic phase (B) to modify the physical and chemical properties of the resultant particle product.

An antigen or bioactive substance may be introduced into either the aqueous phase (A) or the organic phase (B). The organic phase (B) is added into the stirred aqueous phase (A) at a continuous rate. The solvent is evaporated, preferably by a rise in temperature over ambient and/or the use of a vacuum pump. The particles are now present as a suspension (C).

The particles (D) are then separated from the suspension (C) using standard colloidal separation techniques, preferably by centrifugation at high 'g' force, filtration, gel permeation chromatography, affinity chromatography or charge separation techniques. The supernatant is discarded and the particles (D) re-suspended in a washing solution (E) preferably water, salt solution, buffer or organic solvent(s). The particles (D) are separated from the washing liquid in a similar manner as previously described and re-washed, commonly twice.

The particles may then be dried. Particles may then be further processed for example, tabletted, encapsulated or spray dried.

The release profile of the particles formed above may be varied from immediate to controlled or delayed release dependent upon the formulation used and/or desired.

Antigen loading may be in the range 0-90% w/w.

Specific examples include the following:

A PTd (168 μg/ml) or FHA (264 μg/ml) solution was first dispersed in a PVA (mwt = 13000-23000; 98% hydrolysis) solution while stirring at 400 rpm with the temperature set at 25°C. A polymer solution (prepared by dissolving PLGA; 50:50; RG504 supplied by Boehringer Ingelheim into the organic phase) was added slowly into the aqueous phase to form coacervates that hardened following evaporation of the organic solvent. The nanoparticles were then recovered by centrifugation at 15,000 rpm for 30 minutes and washed three times with autoclaved deionised water. The wet pellet was allowed to dry at ambient temperature under a vacuum. Batches having a theoretical loading of 1.2% PTd (RG504) and 1.0% FHA (RG504) were prepared according to Table 3.

**Table 3**

| Batch | 5% w/v PVA soln. | PLGA polymer (g) | Acetone (ml) | Ethanol (ml) | antigen (ml) |
|---|---|---|---|---|---|
| 1.2% PTd | 257.1 | 1.976 | 45 | 5 | 142.9 |
| 1.0% FHA | 243.2 | 1.485 | 33.33 | 4.17 | 56.8 |

Scanning microscopy was employed to assess the nanoparticle morphology and size. The nanoparticles were mounted onto SEM stubs, sputter coated using an Emitech K550 sputter coater set at 25 mA for 3 minutes and scanned using a Leica Cambridge S360. Micrographs were taken at magnifications of 500 - 20,000x. Particle size assessment by SEM was carried out by dividing the micrographs at the 15,000 magnification into different fields and counting the number of particles greater and less than 600 nm and 500 nm. The SEM analysis showed that the nanoparticles were approximately spherical in shape with smooth surfaces. At least 50 % of the particles were less than 600nm at the 15k magnification, although there was some evidence of aggregation

Antigen loading was determined by measuring the total protein content of the nanoparticles using a BCA protein assay as described in Example 1. The nanoparticles prepared according to the present example were found to have the potencies and encapsulation efficiencies as given in Table 5.

**Table 4**

| Batch | Potency (μg/ml) | Encapsulation efficiency (%) |
|---|---|---|
| 1.2% PTd - RG504 | 3.3 | 27.5 |
| 1.0% FHA - RG504 | 4.6 | 45.6 |

The *in vitro* release of antigen from the loaded particles was determined by suspending 50 mg of nanoparticles in 10 ml PBS, pH 7.4, containing 0.02% w/v sodium azide in glass tubes and incubating at 37°C. At predetermined time intervals, a 3 ml sample was removed and the total protein released was determined by the BCA protein assay described above. PTd-PLGA formulations showed a large burst effect of approximately 45% in the first hour followed by a very gradual release up to 55% at 24 hours. In comparison, FHA-PLGA formulations showed a much lower burst release of 14% at 1 hours up to 18% after 24 hours.

Example 5

Immune response upon oral administration of KLH-PLGA particles to balb/c mice

The immunogenicity of KLH entrapped in biodegradable microparticles was assessed in mice following oral immunisation by oral gavage and compared with oral immunisation of the same antigen in solution combined with empty microparticles. Further control groups included soluble antigen alone and empty PLGA microparticles alone. Microparticles from the two batches of Example 1 were pooled and suspended in PBS at a concentration equivalent to 100 μg of antigen per ml or diluted accordingly for lower doses. Each mouse was immunised at weeks 0 and 4 and immune responses were assessed 2 weeks after the final immunisation.

| Table 5 | | |
|---|---|---|
| **Immunogen** | **Responders** | **Serum IgG titre Mean (SD)** |
| KLH-PLGA (100 μg) | 5/8 | 3.30 (0.4) |
| KLH-PLGA (10μg) | 0/4 | NA |
| KLH-PLGA (1 μg) | 0/4 | NA |
| Soluble KLH (100 μg) + empty PLGA | 12/14 | 3.65 (0.5) |
| Soluble KLH (100μg) | 2/10 | 3.15 (0.3) |
| Empty PLGA | 0/14 | NA |

Serum and mucosal secretions (lung homogenates) were tested for anti-KLH IgG and IgA antibody levels by ELISA. Oral immunisation with KLH microparticles generated circulating KLH-specific IgG as shown in Table 3 and low levels of IgA in lung secretions.

Cellular immune responses were assessed using spleen from immunised mice. The spleen cells from 4 to 6 individual mice in each experimental group were cultured in triplicate wells of duplicate 96-well microtitre plates with a range of concentrations of antigen (0.16 to 100μg/ml). The mitogens Concanavlin A or PMA and anti-CD3-antibody or medium alone were included as positive and negative controls respectively. After 24 and 72 hours, supernatants were removed from one plate and stored at -70°C for cytokine analysis. The levels of interferon γ (IFN-γ) and interleukin-5 (IL-5) were determined by immunoassay as quantifiable markers of induction of antigen-specific $T_H1$ and $T_H2$ subpopulations respectively. Additionally, the proliferation of T cell cultures were assessed in four day cultures, by [$^3$H]-thymidine incorporation.

Oral immunisation with 100 μg KLH entrapped in PLGA microparticles induced significant proliferative T cell responses and high nanogram levels of IFN-γ produced by spleen cells following *in vitro* stimulation with KLH over a wide dose range. Picogram levels of IL-5 were also detected in antigen-stimulated spleen cell supernatants but the levels were relatively low and comparable to that observed with spleen cells from animals immunised with soluble KLH in combination with empty microparticles. Overall, the responses were distinctly polarised to $T_H2$ for soluble KLH in combination with empty microparticles and to $T_H1$ with microencapsulated KLH. Fig. 1 shows the immune response following the second immunisation at four weeks. The levels of IL-5 for the microencapsulated KLH were comparable with those observed with soluble antigen + empty microparticles, but the production of IFN-γ was significantly higher.

These results demonstrate that the entrapment of the soluble antigen KLH in PLG microparticles significantly enhanced T cell proliferative responses over that observed with soluble antigen in combination with empty microparticles when administered orally. Moreover, encapsulation of the antigen KLH in PLGA appears to favour the induction of $T_H1$ cells.

The effect of dose was pronounced following immunisation by the oral route. Doses in excess of 10 μg of antigen (in approximately 2.8 mg microparticles) were required to generate detectable immune response. High levels of IFN-γ production and moderate to low levels of IL-5 were detected following antigen stimulation of spleen cells from mice immunised with 100 μg of KLH entrapped in PLG microparticles. In contrast, T cell cytokine production and antibody responses were weak or undetectable following immunisation with 10 or 1 μg of microencapsulated KLH.

Example 6

Immune response upon oral administration of PTd-PLGA particles to balb/c mice.

Similarly to the immunisation regimen of Example 5, batch PTd-1 of Example 2 was used in a oral immunisation study in which groups of 5 balb/c mice were immunised by oral gavage with 100 μg PTd entrapped in PLGA particles, with soluble antigen alone, and with soluble antigen mixed with empty PLGA microparticles. Control mice received empty PLGA microparticles. Two weeks after two immunisations (weeks 0 and 4), mice were sacrificed and serum and lungs were recovered for antibody analysis and spleen for CMI studies. One mouse in the PTd-PLGA group died during the course of the experiment leaving 4 animals in the group.

The results of the T cell proliferative responses, shown in Fig. 2, reveal that each of the 4 mice immunised with PTd entrapped in PLGA microparticles gave a positive proliferative response to PT *in vitro.* The response was relatively strong at the high dose of antigen in all 4 animals and also highly significant (stimulation index > 3) at the lower dose of antigen in 3 of the 4 mice. 2 of 5 mice immunised with soluble PTd and 3 of 5 immunised with soluble PTd combined with empty microparticles showed positive PT-specific responses. One of the 5 mice immunised with empty PLGA also showed a positive response to inactivated PT, but only to the high antigen concentration. Spleen cells from all mice responded to the polyclonal activator PMA and anti-CD3-antibody.

The results of the serum IgG responses, shown in Fig. 3, reveal that 3 out of 4 of the mice immunised with PTd entrapped in PLGA developed a serum antibody response against PT. The end point titres were in the range of 3.5 - 4.5. 2 of 4 mice immunised with soluble PTd and 4 of 5 immunised with soluble PTd combined with empty microparticles showed positive PT-specific antibody responses with titres in excess of 4.0.

Figs. 5 and 6 show the cytokine analysis at week 6 from splenic T cells of mice immunised by oral gavage at week 0 and week 4 with 100 μg PTd entrapped in PLGA, 100 μg soluble PTd in combination with empty PLGA microparticles, 100 μg soluble PTd and with empty PLGA microparticles. Spleen cells from individual mice were stimulated with inactivated PT, *B. pertussis* and with the positive control PMA/anti-CD3-antibody. After 3 days culture supernatants were tested for IL-5 and IFN-γ by specific immunoassays. No explicit $T_H1$ or $T_H2$ polarisation was observed. The use of splenic T cells may render the cytokine response difficult to observe compared, for instance, to the use of Peyer's patches or mesomenteric lymph nodes. However, as discussed above, T cell proliferation and antibody responses were present.

Example 7

Pertussis challenge study following oral immunisation of balb/c mice with the antigen combination PTd +FHA (microparticles)

Groups of 20 balb/c mice were immunised orally 3 times at week 0, week 4 and week 8 with the following formulations: control PLG (empty PLGA microparticles), PTd-PLG (100 μg of PTd entrapped in PLGA microparticles), PTd SOLUTION (100 μg of PTd in solution) and PTd-FHA-PLG (100 μg of each of PTd and FHA entrapped in PLGA microparticles). The ability of PLGA-entrapped antigen to protect against *B. pertussis* was examined in a respiratory challenge model. Briefly, following three doses of antigen, four weeks apart, a respiratory *B. pertussis* infection was initiated in 16 mice per experimental group by aerosol challenge of approximately 2 x $10^{10}$ cfu/ml (approximately $10^4$ - $10^5$ cfu per mouse lung) two weeks after the third immunisation. The mice were sacrificed at different time points over a two-week period and lung homogenates were cultured and examined after 5 days culture for the number of colony forming units (CFU). Four mice from each group were sacrificed prior to challenge to test immune responses on the day of challenge.

The results from the CFU counts 2 hours and 3, 7, 10 and 14 days after challenge are shown in Fig. 6 and reveal a high level of protection with the PLGA microparticle-entrapped combination of PTd and FHA. This treatment provided clearance of *B. pertussis* by day 14 post challenge following challenge 2 weeks after the third immunisation. The formulations containing PLGA entrapped PTd and FHA reduced the CFU counts in the lungs at day 14 over 2 log units compared to the control. While solutions of antigens were also protective, they were less potent than the blend entrapped PLGA microparticle formulation.

Example 8

Immunogenicity and challenge study following oral immunisation of balb/c mice with antigen combination PTd + FHA (coacervated nanoparticles)

Three groups of 21 mice were immunised at 0, 4 and 8 weeks with the following treatments:

Treatment 1: PTd + FHA in saline solution (100 μg of each antigen)
Treatment 2: PTd + FHA in PLGA (blend of 100 μg of each of antigen entrapped in nanoparticles according to Example 4)
Treatment 3: Empty nanoparticles

Antigens were administered by oral gavage in 2 doses of 0.75 ml, with a rest of 1 hour between doses, to mice that had been fasted at least 3 hours.

Immune responses were assessed at week 10. Spleen cells from 5 individual mice, pooled mesenteric lymph node or Peyer's patch were tested for antigen-induced proliferation and cytokine production. Serum and lung homogenates were assessed for anti-PT and anti-FHA IgG and IgA, respectively, on the day of challenge and 3,7,10 and 14 days post challenge (blood samples were removed from 4 mice sacrificed from each group prior to removal of lungs for CFU counts). Mice (16 per groups receiving Treatments 1 and 2; 20 in the control group) were challenged at week 10 according to the respiratory challenge model described in Example 7 and CFU counts were performed on individual lung homogenates (4 mice per group) at 3, 7, 10 and 14 days post challenge. An additional 4 un-immunised control mice were assessed for CFU levels 2-3 hours after challenge to establish the day 0 CFU counts.

Serum IgG antibody responses were assessed at various intervals after challenge as well as on the day of challenge to measure the variability between animals and to examine the possibility of an anamnestic antibody response following oral priming. The overall patterns of the serum IgG response induced following 3 immunisations with PTd and FHA entrapped in PLGA nanoparticles or in solution by the oral route revealed considerable variability between mice, especially the mice immunised with the antigens in solution. In general, similar anti-PT responses were induced with the PLGA nanoparticulate entrapped and soluble pertussis antigens. Although the number of mice responding were similar for both formulations, the mean titres for the FHA-specific serum IgG was significantly stronger in a proportion of the mice immunised with the antigens in solution. The antibody responses in the control group that received empty PLGA nanoparticles remained undetectable up to 14 day post challenge. Anti-FHA antibody titres greater than 3.0 $\log_{10}$ were observed in 12 of 16 mice immunised with antigens in solution and in 13 of 16 mice immunised with PLGA entrapped antigens, whereas anti-PT titres greater than 3.0 were observed in 15 of the 16 mice immunised with either formulation. There is some evidence of an anamnestic antibody response, especially the anti-PT response, post challenge in mice immunised with antigens entrapped in PLGA.

Surprisingly high levels of antigen-specific IgA were observed in the lungs on the day of challenge in mice immunised with PTd and FHA entrapped in PLGA or in solution. Each of the 4 mice examined had IgA titres against PT in the range 2.0 to 3.2. However, the anti-FHA titres were significantly stronger in mice immunised with the antigens in solution.

Significant PT- and FHA-specific T cell proliferation was observed in individual spleen cells from 3of 4 mice immunised with PTd and FHA entrapped in PLGA. Similar responses were observed in mice immunised with antigen in solution. Spleen cells from control mice gave background proliferation except against the polyclonal activators (PMA/anti-CD3) and the higher dose of killed bacteria. An examination of the cytokine production shows that spleen cell preparations from each of the 4 mice immunised with PTd and FHA in solution or entrapped in PLGA secreted relatively high levels (greater than 500 pg/ml) of IL-5 in response to stimulation with FHA, PT or killed *B. pertussis in vitro.* Although the levels were not consistently positive with all mice and all antigen preparations, IFN-γ was detected in spleen cells from mice immunised with either formulation. However, there was some background response, especially to the killed *B. pertussis* and high doses of FHA in control mice immunised with empty PLGA nanoparticles. In contrast, the antigen-stimulated IL-5 was undetectable in the mice receiving empty PLGA nanoparticles. The overall pattern was $T_H2$ or mixed $T_H1/T_H2$ with greater polarisation to $T_H2$ for antigens in solution or entrapped in PLGA nanoparticles given by the oral route.

Mesenteric lymph nodes, pooled from mice immunised with PTd and FHA entrapped in PLGA or in solution responded in a proliferation assay to FHA and killed bacteria. Cells from control mice responded to killed bacteria and not to FHA. Significant levels of IL-5 were detected in supernatants of mesenteric lymph nodes cells from immunised mice following *in vitro* stimulation with FHA and to a lesser extent with PT. In contrast cells, from unimmunised control mice only produced IL-5 in response to PMA and anti-CD3. IFN-g was detected in supernatants of mesenteric lymph nodes in response to killed *B. pertussis.* However, similar levels were detected in immunised and unimmunised control mice and IFN-γ was undetectable following stimulation with purified FHA or PT. These results indicate that oral immunisation with pertussis antigens in solution or entrapped in PLGA nanoparticles induce a $T_H2$ response in the mesenteric lymph nodes.

The T cell responses of pooled Peyer's patch cells was generally very weak Although significant levels of proliferation, IL-5 and IFN-γ were detected against PMA and anti-CD3 and low levels against killed *B. pertussis* in all experimental groups, PT and FHA included T cell activation was weak or undetectable.

Mice were challenged 2 weeks after the third immunisation. A rapid initial drop in CFU counts was observed in mice immunised with FHA and PTd entrapped in PLGA nanoparticles (Figure 7). At 3 days, the CFU counts were 1.5 logs lower than in the mice immunised with the antigens in solution and more than 3 logs lower than the controls. A typical rebound in the CFU counts is observed at day 7. The overall protection with the PLGA entrapped pertussis antigens appears to be significantly better than with the antigens in solution. Assigning a potency index to the protection according to the formula describe in Mills, et al. Dev. Biol. Std. 95:31-41 (1998), values of 62.8 and 44.8 can be assigned to the PLGA entrapped and soluble antigens, respectively. Extrapolation from the correlation curve translates to 73% and 48% efficacy in children. They reveal a high level of protection in animals orally immunised with a blend of nanoparticles entrapping PTd and FHA respectively. While soluble antigens were also protective, the clearance was less effective than the PLG formulation at each timepoint. The efficacy of the nanoparticle entrapped FHA and PTd is roughly comparable with that observed for the solvent evaporated microparticles delivered by the oral route according to Example 7 (67% efficacy in children).

Example 9

Preparation of KLH-PLA microparticles by a spray drying method (comparison example)

A PLA (poly D, L lactide; molecular weight 16,000 solution; i.v. = 0.27 dl/g; supplied by Boehringer Ingelheim, R203) solution in ethylacetate (5 % PLA in 150 ml ethylacetate) was prepared two hours prior to use. The polymer solution was homogenised (at 24,000 rpm) using an IKA Ultra Turrax T25 homogeniser with an S1 head while the KLH antigen solution (62 mg KLH in MES (2-[n-morphlino] ethanesulfonic acid)) was added slowly. The emulsion was cooled on ice and homogenisation was continued for 1 min. The single emulsion thus prepared was spray dried using a Büchi 191 mini spray drier with continuous stirring using a magnetic stirrer. The following parameters were used in the spray drying step:

**Table 6**

| Parameter | Value | Parameter | Value |
|---|---|---|---|
| Inlet temperature (°C) | 60 | Aspirator (%) | 100 |
| Outlet temperature (°C) | 45 | Pump rate* (ml / min.) | 5 |
| Flow rate | 700 - 800 | Pressure (mbar) | - 40 |

*Pump rate was set at 25 %, the actual rate of solvent pumped through varied from 5 to 6 ml / min.

The particles were collected immediately from both the collection vessel and the cyclone. The antigen-loaded microparticles were characterised according to the procedures outlined above for the previous examples. The microparticles formed according to the present invention (yield 33%) were found to be smooth and spherical in nature. The loading (μg/mg) was 7.4 with an entrapment efficiency of 91 % and a D50% of 4.6 by laser light diffractometry. The *in vitro* release of KLH was found to be 10% within one hour and 49% on day 23.

Similar to the Examples above, mice were immunised with two oral inoculations (week 0 and week 4) of 100 μg KLH either entrapped in PLA microparticles or in solution (soluble KLH). These particles did not prove immunogenic when administered orally, perhaps due to the slower release profile or the larger particle size compared to the microparticles of Example 1 or due to possible degradation of KLH during the spray dying process.

Example 10

Preparation of PTd-PLA microparticles by a spray drying method (comparison example)

PTd-PLA microparticles were formed by a spray drying method similar to that of Example 9 with the exceptions that, to prevent phase separation during spray drying, the homogenisation speed was increased to 24,000rpm, the polymer viscosity was increased by using 5% R203 and the w/o emulsion was stirred during spraying. The release profile of the resultant particles was characterised by a marked burst of 50-60% in the first hour followed by a very slow release phase over a three month period of time.

Serum anti-PTd IgG levels were determined in mice immunised orally with 100 μg PTd in spray dried PLA microparticles and compared to immunisation with soluble PTd in combination with empty PLA microparticles, empty PLA microparticles and PTd in solution. However, no antibody or T-cell responses were obtained in mice immunised orally with PTd in spray dried PLA microparticles.

The integrity of PTd, FHA and KLH following either the solvent evaporation (*see* Examples above) or spray drying processes were examined semi-quantitatively by PAGE gel analysis. More PTd remains intact when extracted from particles prepared by the solvent evaporation method relative to spray dried particles. Thus, the PTd may have been partially degraded during the spray drying process. The data suggest that while spray-drying is problematic for maintaining antigenic structure in the case of PTd, this cannot be assumed for less labile antigens and it will therefore need to be assessed on a case by case basis.

## Claims

1. A vaccine formulation for oral administration comprising a pharmaceutically acceptable carrier and a pharmaceutically effective amount of microparticles sized such that at least 50% of the microparticles are less than 5 μm, the microparticles comprising at least one antigen entrapped or encapsulated by a biodegradable polymer.

2. The vaccine formulation of Claim 1, wherein the microparticles are sized such that at least 50% of the microparticles are less than 3 μm.

3. The vaccine formulation of Claim 1, wherein the biodegradable polymer comprises a copolymer of lactic acid and glycolic acid or enantiomers thereof.

4. The vaccine formulation of Claim 1, wherein the microparticles are formed using a solvent evaporation method.

5. The vaccine formulation of Claim 1, wherein the antigen comprises a *B. pertussis* antigen.

6. The vaccine formulation of Claim 1, wherein the microparticles comprise at least 2 subpopulations of microparticles, each subpopulation comprising a different antigen entrapped or encapsulated by a biodegradable polymer.

7. A vaccine formulation for oral administration comprising a pharmaceutically acceptable carrier and a pharmaceutically effective amount of nanoparticles sized such that at least 50% of the nanoparticles are less than 600nm, the nanoparticles comprising at least one antigen entrapped or encapsulated by a biodegradable polymer.

8. The vaccine formulation of Claim 7, wherein the nanoparticles are sized such that at least 50% of the microparticles are less than 500nm.

9. The vaccine formulation of Claim 7, wherein the biodegradable polymer comprises a copolymer of lactic acid and glycolic acid or enantiomers thereof.

10. The vaccine formulation of Claim 7, wherein the nanoparticles are formed using a coacervation method.

11. The vaccine formulation of Claim 7, wherein the antigen comprises a *B. pertussis* antigen.

12. The vaccine formulation of Claim 7, wherein the nanoparticles comprise at least 2 subpopulations of nanoparticles, each subpopulation comprising a different antigen entrapped or encapsulated by a biodegradable polymer.

13. Use of a pharmaceutically effective amount of nanoparticles sized such that at least 50% of the nanoparticles are less than 600nm, the nanoparticles comprising at least one *B. pertussis* antigen entrapped or encapsulated by a biodegradable polymer, in the manufacture of a medicament for use in inducing a protective immune response against *B. pertussis* in a subject and wherein the nanoparticles are orally administered to said subject.

14. Use according to Claim 13, wherein the nanoparticles are sized such that at least 50% of the microparticles are less than 500nm.

15. Use according to Claim 13 or 14, wherein the biodegradable polymer comprises a copolymer of lactic acid and glycolic acid or enantiomers thereof and wherein the nanoparticles are formed using a coacervation method.

16. Use according to any one of Claims 13-15, wherein the at least one *B. pertussis*_antigen is selected from the group consisting of inactivated pertussis toxin (PTd), filamentous hemaglutinin (FHA), pertactin and fimbrae and combinations thereof.

Fig. 1

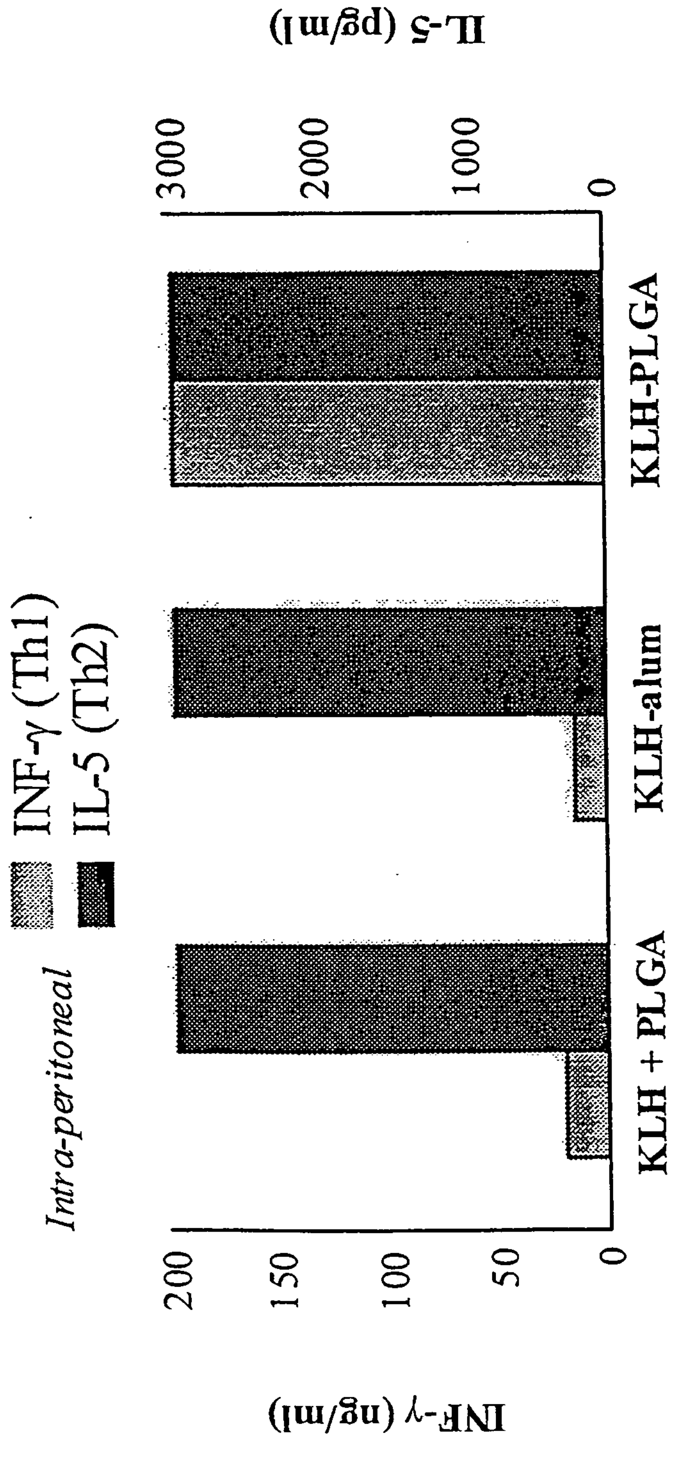
Intra-peritoneal
INF-γ (Th1)
IL-5 (Th2)
KLH + PLGA
KLH-alum
KLH-PLGA
INF-γ (ng/ml)
0
50
100
150
200
IL-5 (pg/ml)
0
1000
2000
3000

Fig. 2

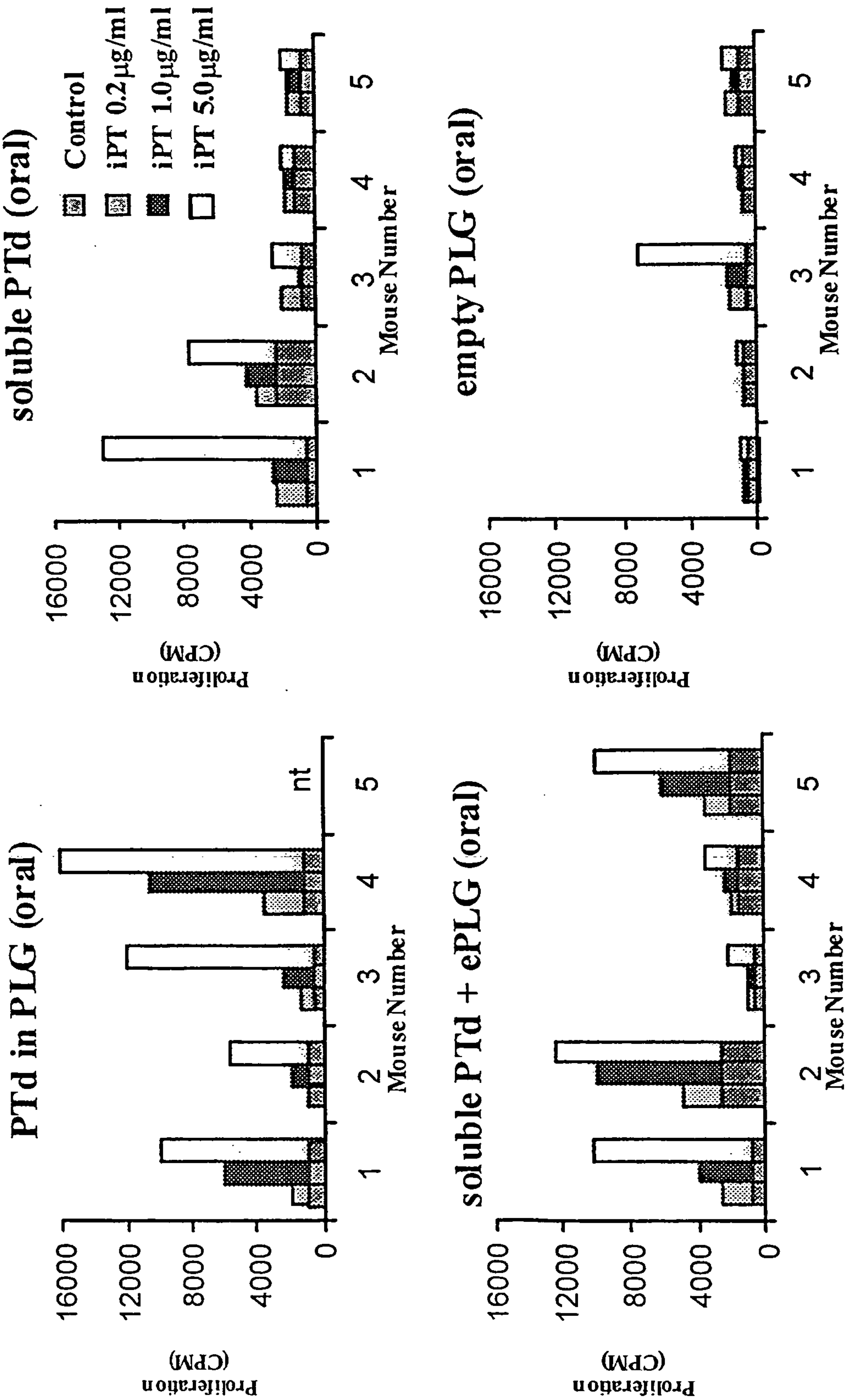
PTd in PLG (oral)
soluble PTd (oral)
soluble PTd + ePLG (oral)
empty PLG (oral)
Control
iPT 0.2µg/ml
iPT 1.0µg/ml
iPT 5.0µg/ml
Proliferation (CPM)
Mouse Number
nt
16000
12000
8000
4000
0
1
2
3
4
5

Fig. 3

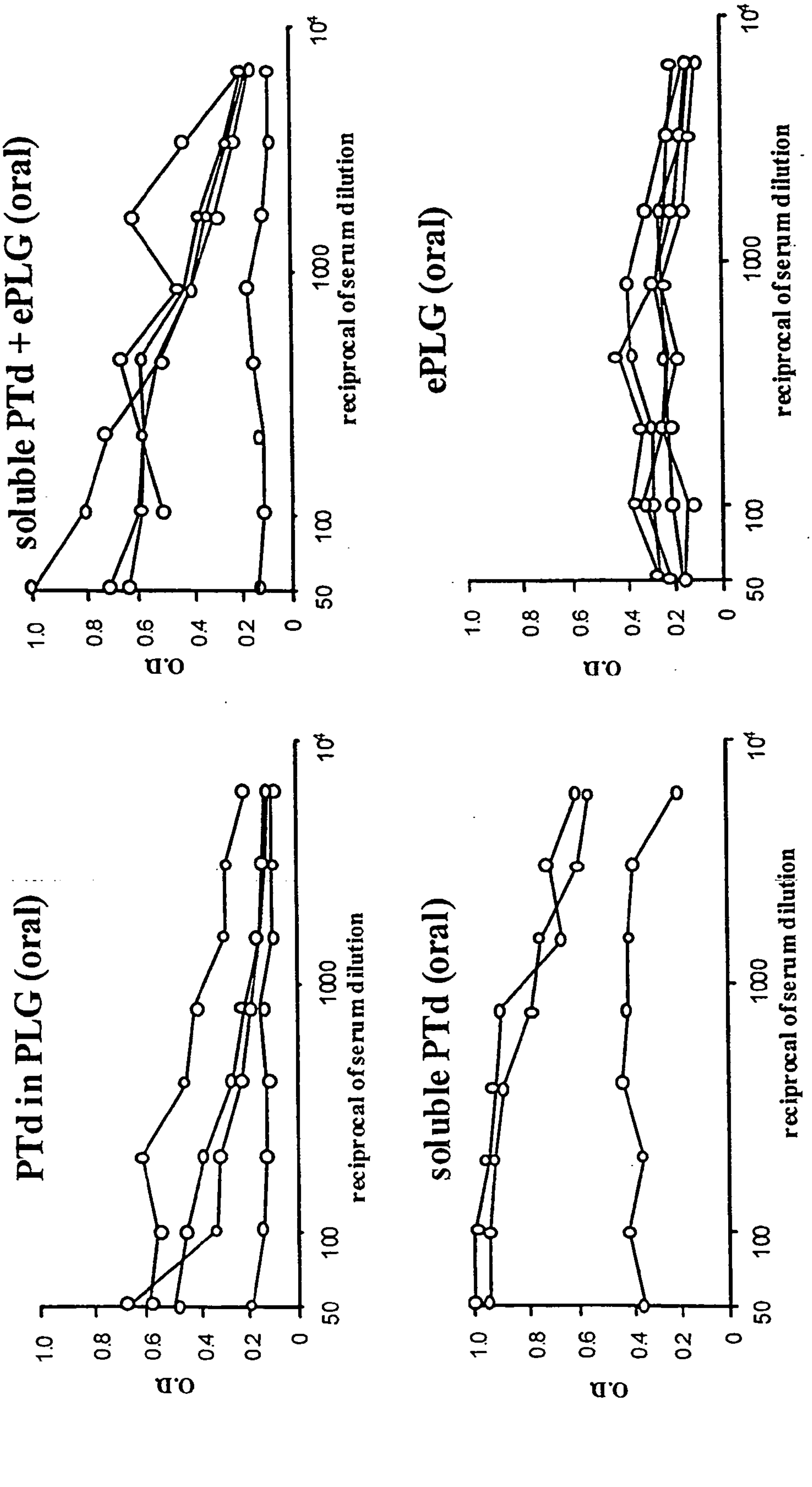
PTd in PLG (oral)
soluble PTd + ePLG (oral)
soluble PTd (oral)
ePLG (oral)
O.D
1.0
0.8
0.6
0.4
0.2
0
50
100
1000
$10^4$
reciprocal of serum dilution

Fig. 4

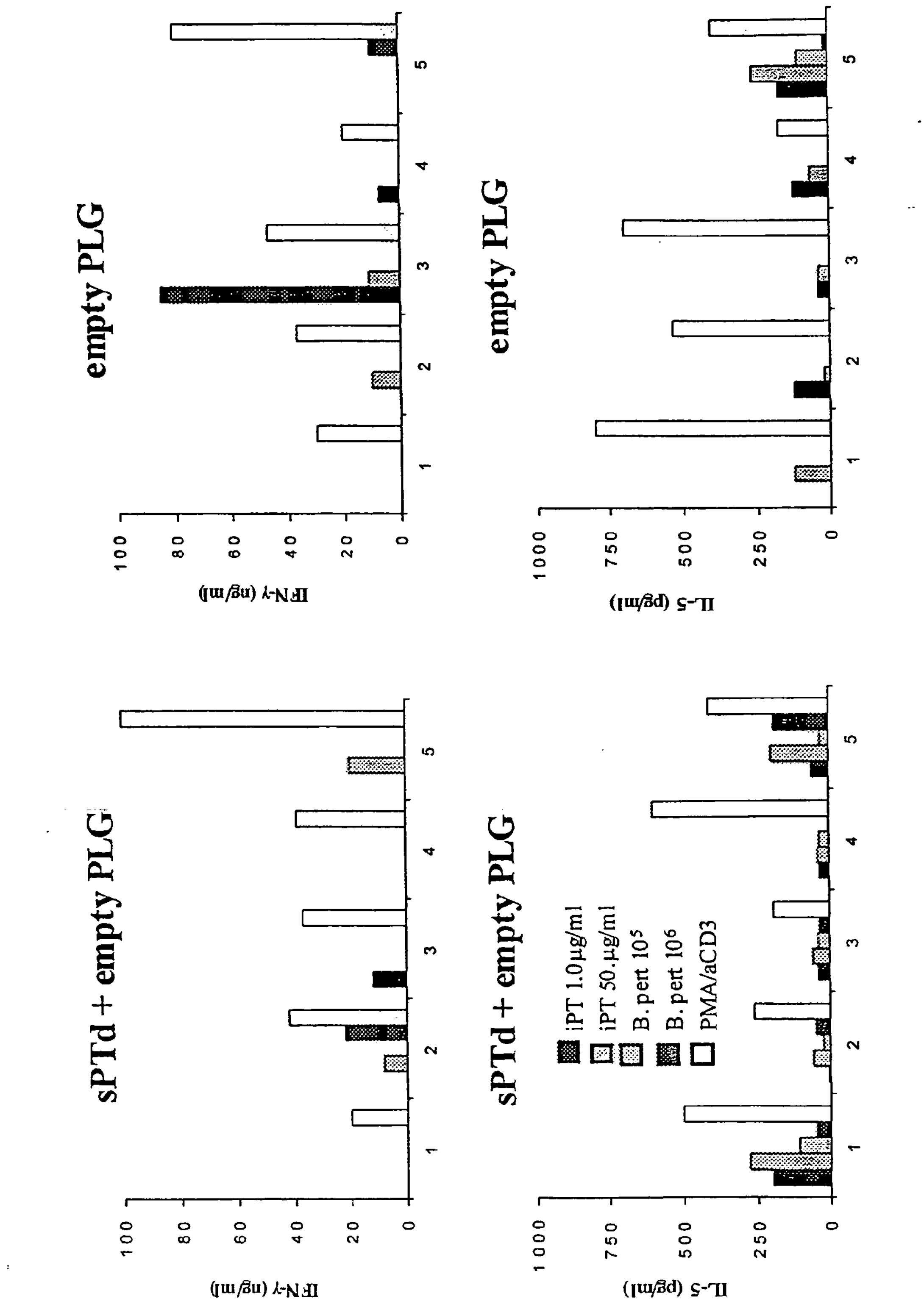
empty PLG
IFN-γ (ng/ml)
IL-5 (pg/ml)
sPTd + empty PLG
iPT 1.0μg/ml
iPT 50.μg/ml
B. pert 10⁵
B. pert 10⁶
PMA/aCD3

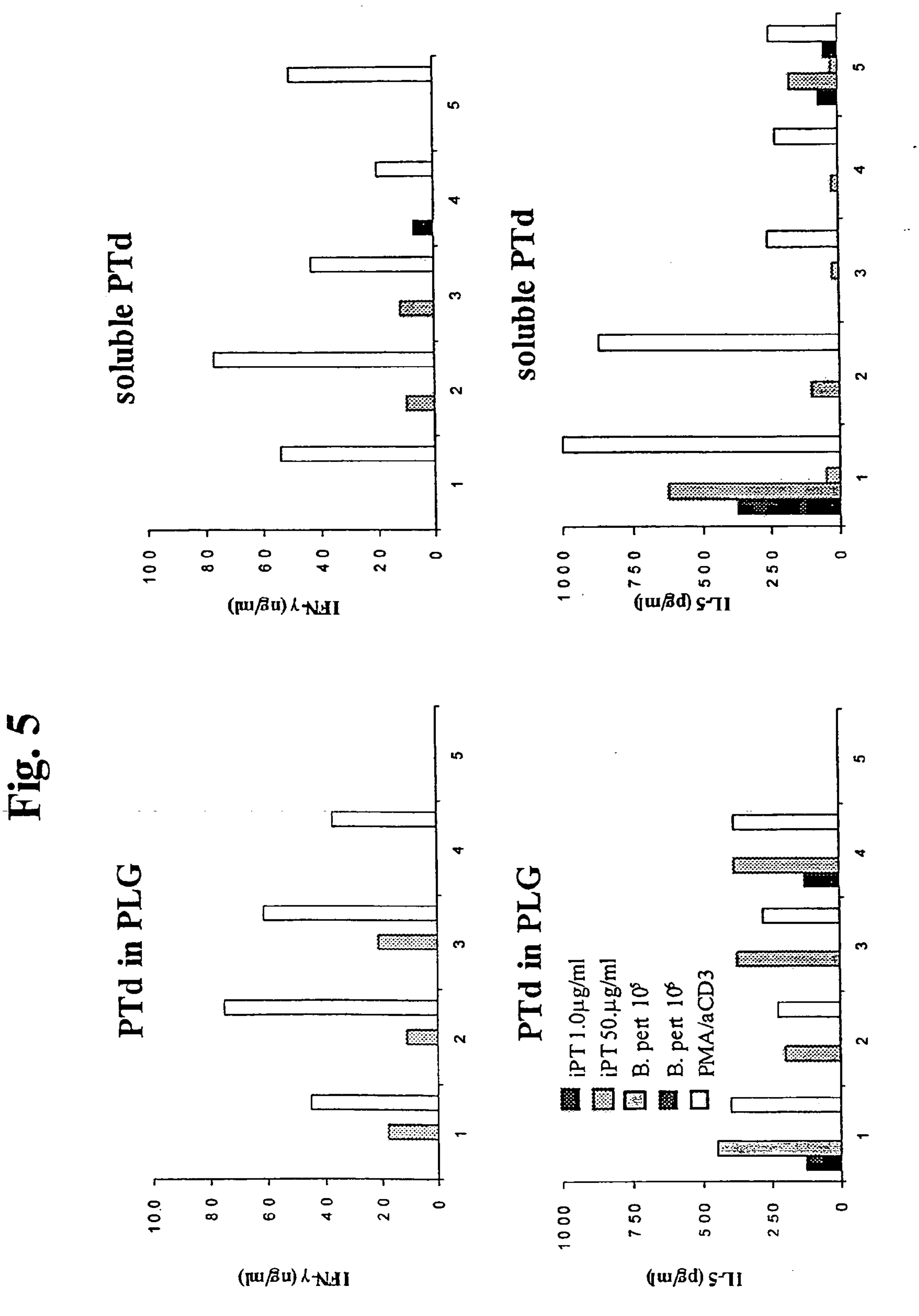
Fig. 5
PTd in PLG
soluble PTd
IFN-γ (ng/ml)
IL-5 (pg/ml)
iPT 1.0µg/ml
iPT 50µg/ml
B. pert 10^5
B. pert 10^6
PMA/aCD3

Fig. 6

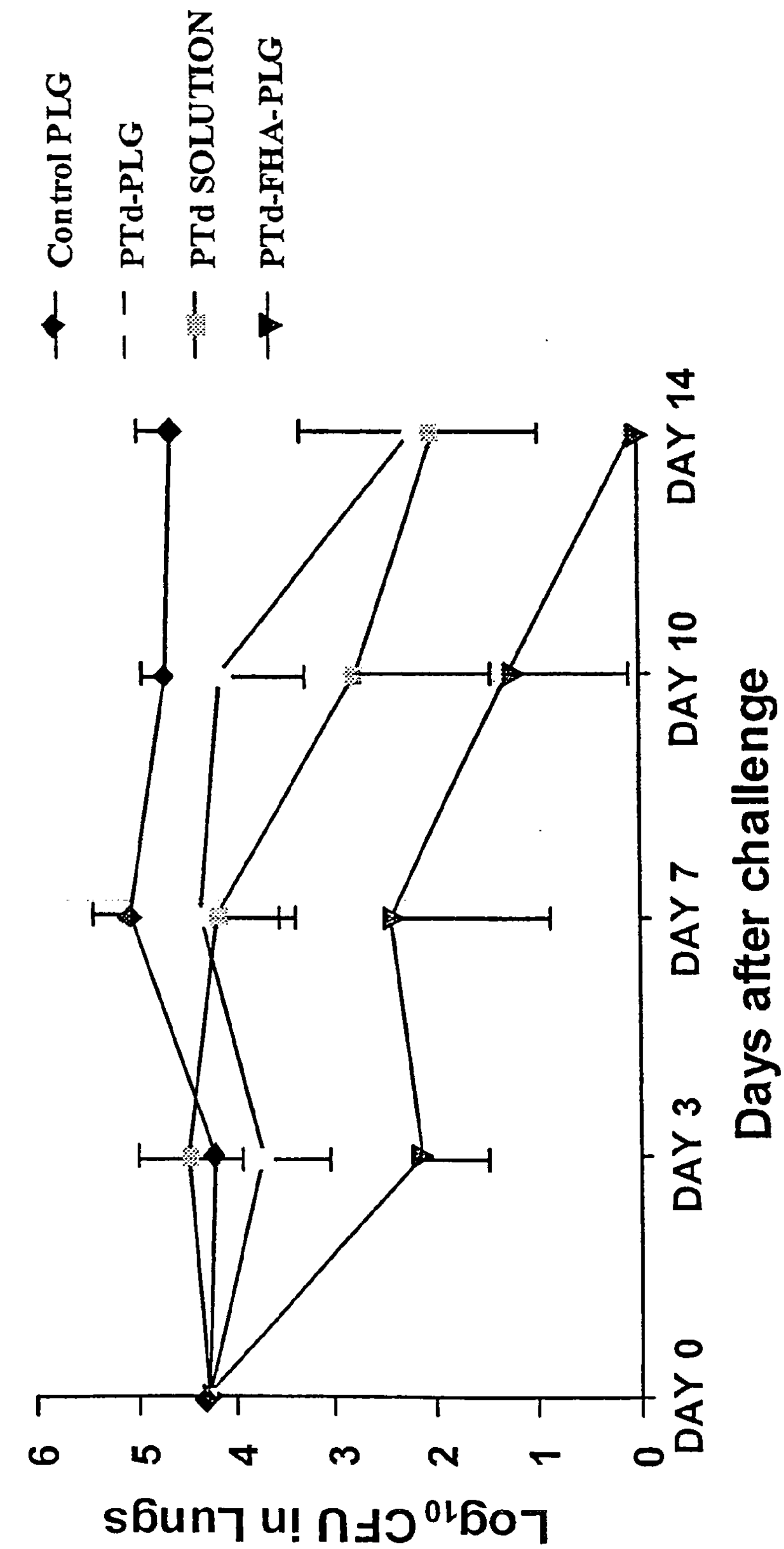

Control PLG
PTd-PLG
PTd SOLUTION
PTd-FHA-PLG
$Log_{10}$ CFU in Lungs
0
1
2
3
4
5
6
DAY 0
DAY 3
DAY 7
DAY 10
DAY 14
Days after challenge

Fig. 7

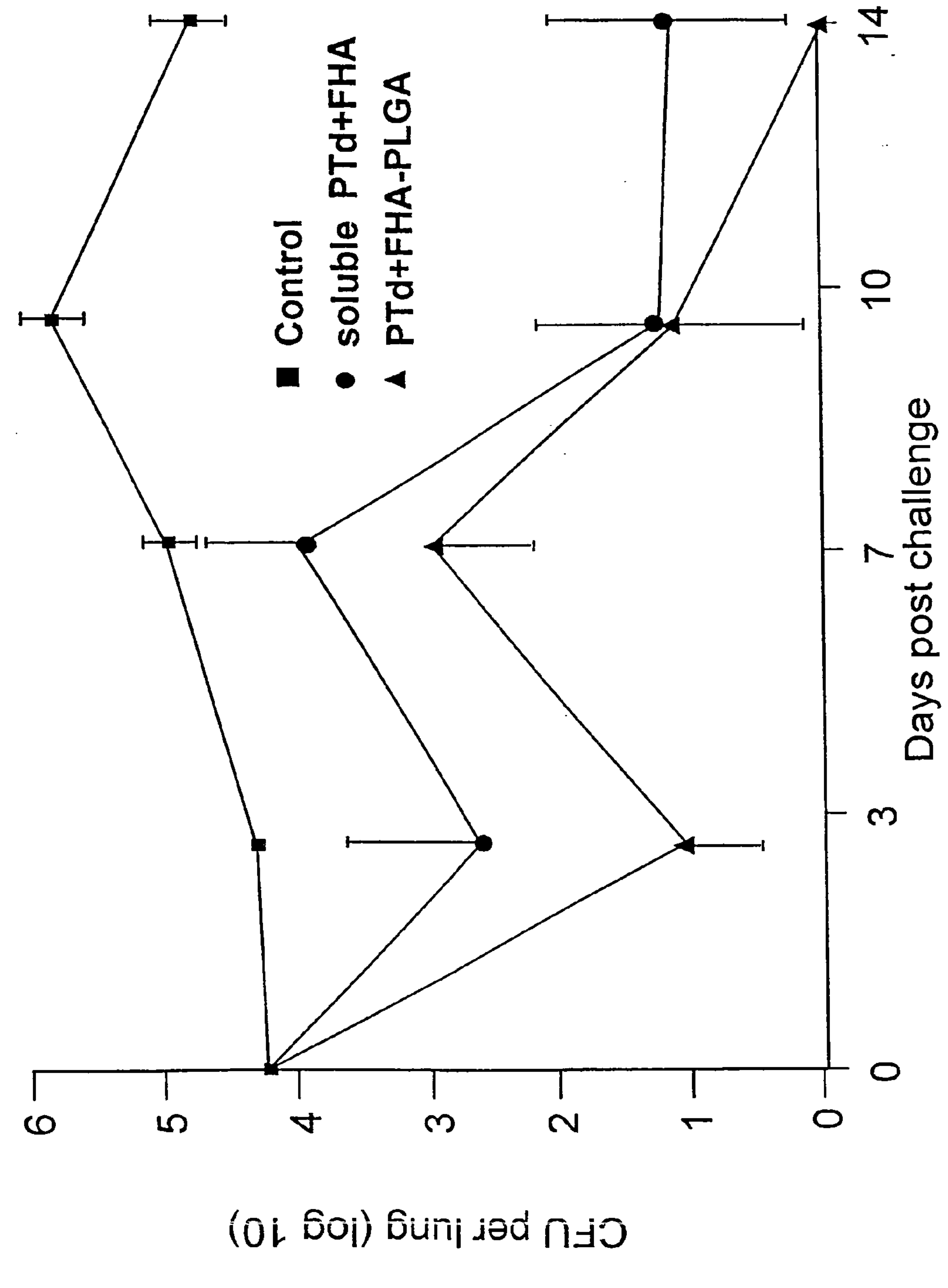
Control
soluble PTd+FHA
PTd+FHA-PLGA
CFU per lung (log 10)
Days post challenge
0
1
2
3
4
5
6
0
3
7
10
14

Europäisches Patentamt
European Patent Office
Office européen des brevets

# EUROPEAN SEARCH REPORT

Application Number
EP 09 01 3789

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | SHAHIN R, ET AL.: "Adjuvandicity and protective immunity elicited by Bordetella pertussis antigens encapsulated in poly(DL-lactide-co-glycolide) microspheres" INFECTION AND IMMUNITY, vol. 63, no. 4, 1995, pages 1195-1200, XP002124771 ISSN 0019-9567 * abstract * * page 1196, left-hand column, last paragraph - right-hand column * * page 1198, right-hand column, line 5 - line 19; table 7 * * page 1199, right-hand column, line 59 - page 1200 * ----- | 1-6 | INV. A61K39/00 A61K9/16 A61K9/51 A61K39/10 A61K9/00 |
| X | WO 96/20698 A (UNIV MICHIGAN ;LEVY ROBERT J (US); LABHASETWAR VINOD D (US); SONG) 11 July 1996 (1996-07-11) * page 6, line 13 - page 7, line 7 * * page 10, line 14 - page 11, line 2 * * page 24, line 19 - page 26, line 4 * * examples 5,10; table 4 * * page 105, line 5 - page 108, line 9; figure 21; examples 21-23 * * claims 1-7,12,19,20,39,40,44 * ----- -/-- | 1-16 | |
| | | | TECHNICAL FIELDS SEARCHED (IPC) |
| | | | A61K |

The present search report has been drawn up for all claims

1

| Place of search | Date of completion of the search | Examiner |
|---|---|---|
| The Hague | 17 February 2010 | Epskamp, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 01 3789

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | JONES DH, ET AL.: "Orally administered microencapsulated Bordetella pertussis fimbriae protect mice from B. pertussis respiratory infection" INFECTION AND IMMUNITY, vol. 64, no. 2, 1996, pages 489-494, XP002094244 ISSN 0019-9567 * abstract * * page 489, right-hand column, last line - page 490, left-hand column, paragraph 3 * * page 490, right-hand column, paragraph 2 - page 491, left-hand column * ----- | 1-6 | |
| X,D | CAHILL ES, ET AL.: "Immune responses and protection against Bordetella pertussis infection after intranasal immunization of mice with filamentous haemagglutinin in solution or incorporated in biodegradable microparticles" VACCINE, vol. 13, no. 5, 1995, pages 455-462, XP004057720 ISSN 0264-410X * abstract * * page 456, left-hand column, line 56 - right-hand column, line 44 * * page 460, left-hand column, line 30 - right-hand column, line 2 * ----- | 1-6 | TECHNICAL FIELDS SEARCHED (IPC) |
| A,D | WO 93/21950 A (MEDEVA HOLDINGS BV ;ROBERTS MARK (GB); DOUGAN GORDON (GB)) 11 November 1993 (1993-11-11) * page 5, line 8 - page 7, line 16 * * claims 1-3,5,6,9,10 * ----- -/-- | 1-16 | |

The present search report has been drawn up for all claims

1

| Place of search | Date of completion of the search | Examiner |
|---|---|---|
| The Hague | 17 February 2010 | Epskamp, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 01 3789

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DESAI MP, ET AL.: "Gastrointestinal uptake of biodegradable microparticles: effect of particle size" PHARMACEUTICAL RESEARCH, vol. 13, no. 12, 1996, pages 1838-1845, XP000862849 ISSN 0724-8741 * abstract * * page 1838, right-hand column, line 46 - page 1839, left-hand column, line 15; table 1 * * figure 3; table 2 * * page 1842, right-hand column, line 20 - page 1844 * ----- | 1-4, 6-10,12 | |
| A | NUGENT J, ET AL.: "Design and delivery of non-parenteral vaccines" JOURNAL OF CLINICAL PHARMACY AND THERAPEUTICS, vol. 23, no. 4, August 1998 (1998-08), pages 257-285, XP000862868 ISSN 0269-4727 * abstract * * page 258, right-hand column, line 27 - page 260, left-hand column, line 15; figure 1 * * page 261, left-hand column, line 34 - page 262, right-hand column, line 42; tables 2,3 * * page 262, right-hand column, line 43 - page 267, left-hand column; table 4 * * page 276, left-hand column, line 42 - page 277 * ----- | 1-16 | TECHNICAL FIELDS SEARCHED (IPC) |

-/--

1

The present search report has been drawn up for all claims

| Place of search | Date of completion of the search | Examiner |
|---|---|---|
| The Hague | 17 February 2010 | Epskamp, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 01 3789

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | REYNOLDS JEF (ED.): "Martindale, The Extra Pharmacopoeia, Thirty-first Edition" 1996, ROYAL PHARMACEUTICAL SOCIETY, LONDON , ISBN 0-85369-342-0 , XP002124772 * "Pertussis Vaccines"; page 1635 - page 1637 * ----- | 1-16 | |
| X,P | WO 98/58668 A (FARRAR GRAHAM HENRY ;MICROBIOLOGICAL RES AUTHORITY (GB); JONES DAV) 30 December 1998 (1998-12-30) * claims 1-6,13-18; example 5 * ----- | 1-5 | |
| A | MARX P A ET AL: "PROTECTION AGAINST VAGINAL SIV TRANSMISSION WITH MICROENCAPSULATED VACCINE" SCIENCE, AMERICAN ASSOCIATION FOR THE ADVANCEMENT OF SCIENCE, WASHINGTON, DC; US, vol. 260, 28 May 1993 (1993-05-28), pages 1323-1327, XP001208139 ISSN: 0036-8075 * note 19; page 1326 * ----- | 1-16 | TECHNICAL FIELDS SEARCHED (IPC) |

The present search report has been drawn up for all claims

1

| Place of search | Date of completion of the search | Examiner |
|---|---|---|
| The Hague | 17 February 2010 | Epskamp, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 01 3789

This annex lists the patent family members relating to the patent documents cited in the above-mentioned European search report.
The members are as contained in the European Patent Office EDP file on
The European Patent Office is in no way liable for these particulars which are merely given for the purpose of information.

17-02-2010

| Patent document cited in search report | Publication date | Patent family member(s) | Publication date |
|---|---|---|---|
| WO 9620698 A | 11-07-1996 | AT 252894 T | 15-11-2003 |
| | | AU 4755696 A | 24-07-1996 |
| | | CA 2207961 A1 | 11-07-1996 |
| | | DE 69630514 D1 | 04-12-2003 |
| | | EP 0805678 A1 | 12-11-1997 |
| | | JP 10511957 T | 17-11-1998 |
| WO 9321950 A | 11-11-1993 | AT 186218 T | 15-11-1999 |
| | | AT 340589 T | 15-10-2006 |
| | | AU 4267093 A | 29-11-1993 |
| | | DE 69326948 D1 | 09-12-1999 |
| | | DE 69326948 T2 | 16-03-2000 |
| | | DE 69334066 T2 | 03-05-2007 |
| | | DK 639081 T3 | 10-04-2000 |
| | | DK 0937462 T3 | 29-01-2007 |
| | | EP 0639081 A1 | 22-02-1995 |
| | | ES 2141765 T3 | 01-04-2000 |
| | | ES 2273391 T3 | 01-05-2007 |
| | | GR 3032381 T3 | 27-04-2000 |
| | | PT 639081 E | 28-04-2000 |
| | | US 6562352 B1 | 13-05-2003 |
| WO 9858668 A | 30-12-1998 | AU 731216 B2 | 29-03-2001 |
| | | AU 8119498 A | 04-01-1999 |
| | | CA 2294348 A1 | 30-12-1998 |
| | | EP 1005367 A2 | 07-06-2000 |
| | | JP 2002508761 T | 19-03-2002 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9415636 A
- WO 9511008 A
- EP 0266119 A
- EP 0333523 A
- EP 0706792 A
- EP 0686030 A
- US 5417986 A
- WO 9321950 A, Roberts and Dougan


### Non-patent literature cited in the description


- **Preis et al.** *J. Immunol. Methods,* 1979, vol. 28, 193-197
- **Eldridge et al.** *J. Controlled Release,* 1990, vol. 11, 205-214
- **Moore et al.** *Vaccine,* 1995, vol. 18, 1741-1749
- **Maloy et al.** *Immunology,* 1994, vol. 81, 661-667
- **Newman et al.** *J. Controlled Release,* 1998, vol. 54, 49-59
- **Men et al.** *Vaccine,* 1995, vol. 13, 683-689
- **Shahin et al.** *Infection and Immunity,* 1995, vol. 63, 1195-1200
- **Jones et al.** *Infection and Immunity,* 1996, vol. 64, 489-494
- **Cahill et al.** *Vaccine,* 1995, vol. 13, 455-462
- **Singh et al.** *Vaccine,* 1998, vol. 16, 346-352
- **Mills et al.** *Infection and Immunity,* 1998, vol. 66, 594-602
- **Mills et al.** *Infection and Immunity,* 1993, vol. 61, 399-410
- **Ryan et al.** *Immunology,* 1998, vol. 93, 1-10
- **Mills et al.** *Dev. Biol. Std.,* 1998, vol. 95, 31-41